# EUROPEAN PATENT SPECIFICATION

(11) **EP 2 384 169 B1**
(45) Date of publication and mention of the grant of the patent: **18.09.2013**
(21) Application number: 10700060.6
(22) Date of filing: 05.01.2010
(51) Int. Cl.: A61F 5/00

(54) **CHEST SUPPORT BELT**
BRUSTSTÜTZGÜRTEL
CEINTURE THORACIQUE

(30) Priority: 05.01.2009 NL 2002396
(43) Date of publication of application: 09.11.2011
(73) Proprietor: MMID Products B.V., 2611 AZ Delft (NL)
(72) Inventor: MAGERMANS, Marcel, Peter, NL-2498 AP Den Haag (NL)
(74) Representative: Nederlandsch Octrooibureau
(86) International application number: PCT/NL2010/050002
(87) International publication number: WO 2010/077144

(56) References cited:
- EP-A- 0 260 351
- EP-A- 0 368 583
- WO-A-2008/041688
- GB-A- 709 388
- US-A1- 2001 034 498

## Description

### Field of the invention

The invention relates to a chest support belt, which can be worn around the chest of a patient that has undergone surgery to the chest. When a patient undergoes surgery to the chest, such as for instance heart surgery, the ribs of the patient may need to be cut along the sternum and be re-attached using wire, after completion of the heart surgery. The resulting trauma will need to cure over the period of several weeks. During this time, specific movements of the patient such as getting into an upright position, getting out of bed or sneezing, are very painful as the chest injury is placed under stress.

### Background of the invention

In order to prevent a painful expansion of the chest, a chest immobilising support belt is known from EP 0 260 351, wherein a harness is described having a belt that partly encircles the chest. The belt is supported by shoulder straps on the patient and has two free ends, each with a handle that can be gripped by a patient with one hand in order to tighten the belt and to relieve the stress from the chest.

The known chest immobilising strap has as a disadvantage that the position of the end parts of the belt is not defined and that a separate shoulder strap is required for properly positioning the strap on the patient. This results in a strap that is difficult to apply and to a large degree of visibility of the strap, which may negatively affect the appearance of the patient. Furthermore, as the ends of the strap are free to move independently, the pull direction of the known strap is not defined, such that a restricting squeezing force may not be applied in an effective as possible manner.

A chest support belt as defined in the preamble of claim 1 is disclosed in GB-A-709 388.

Accordingly, it is an object of the invention to provide a chest support strap which may be easily attached around the chest of a patient and that can be operated in an easy one-handed manner. It is a further object of the invention to provide a strap that can be worn inconspicuously by the patient. It is again an object to provide a strap that can be fixed in a squeezing position and that can be quickly released.

### Summary of the invention

Hereto the chest support belt according to the invention comprises a strap that is, in use, connected with a first end to a connector end part of a base member, a second end of the strap being attached to a sliding grip and being guided from a guide end part of the base member, which guide end part is situated opposite to the connector end part, over a guide surface of the base member, in the direction of the connector end part to a roller member, and along the roller member back in the direction of the guide end part, wherein the sliding grip can slide along the base member, the base member comprising near the guide end part a fixed grip that is fixed to the base member, which fixed grip in an untensioned state of the strap is situated at a distance of between 5 and 25 cm, preferably between 10 and 20 cm from the sliding grip.

By attaching both ends of the strap and guiding one end via a loop on the base member back to wards the guide end part of the gripping member, the patient can easily place the strap around the chest in an untensioned state, and can by one-handed gripping the sliding grip and the fixed grip on the base member, squeeze his fingers towards his palm to tighten the strap by pulling it around the roller member along the base member. In this manner, a defined pull force can be exerted on the chest. When the strain on the chest has disappeared, for instance after sneezing, the patient can release the gripping parts of the strap which can then relax and expand back to its larger circumferential dimension. By using a closed strap around the chest of the patient, the belt of the present invention can remain in its proper position without the need for additional shoulder straps.

In one embodiment, the sliding grip comprises a slit through which the strap is guided, the slit having about the same width as the width of the strap. By sliding the grip over the strap, the strap functions as a rails and the width of the grip can remain about the same as the width of the strap. Furthermore, guiding the sliding grip over the strap instead of along the base member section, provides a fail safe and simple mechanism, which avoids jamming and tilting of the base member. The strap may be guided through a second slit in the base member from the guide end part towards the roller member.

For easy coupling of the chest support belt, the base member may near the connector end part have a first connector member, which cooperates with a second connector member attached to the first end of the strap. The strap can be fixed around the chest in an easy and low-force manner.

In order to be able to keep the belt in a tensioned state and using both hands, the base member of a chest support belt according to the invention may have a pivoting lever near the roller member, which lever can be pivoted with a clamping surface into clamping engagement with the roller member.

To keep the base member clear from the underlying wound, the base member may have a curved surface with the first and second ends defining a support surface and with a middle section spaced at a distance from the support surface.
Fig. 1 shows a three-dimensional view of the chest support belt according to the invention in a detached state,
Fig. 2 shows a cross-sectional detail of the base member and grips of the support belt shown in fig. 1,
Fig. 3 shows a perspective view of the base member and connector members in the closed state,
Fig. 4 schematically shows the tensioning operation of the chest support belt after applying the belt to the chest of a patient,
Fig. 5 schematically shows the locking function of the chest support belt of the present invention.

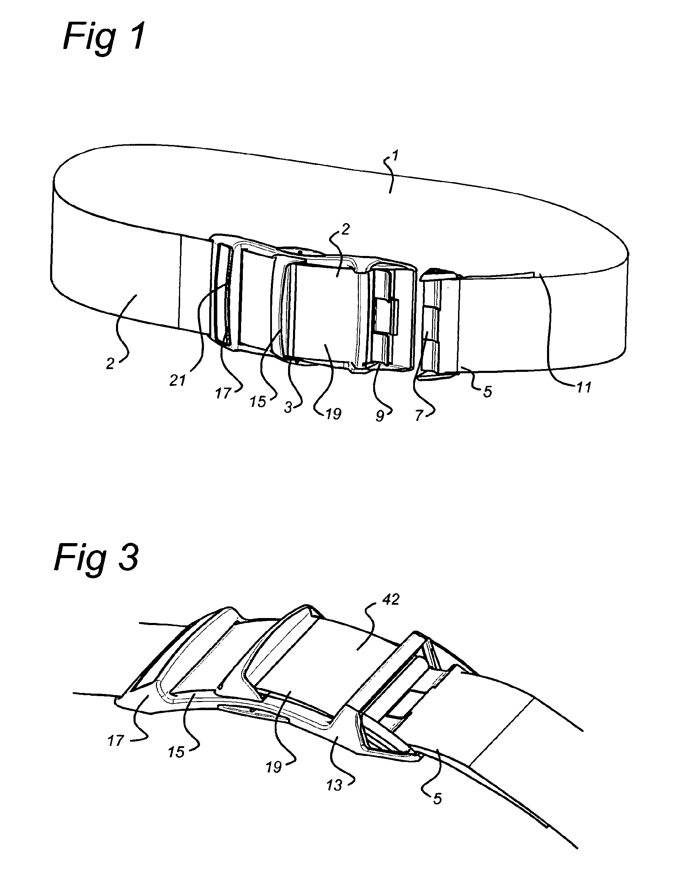

Fig 1 shows a chest support belt 1 having a strap 2 and a base member 3. The strap 2 has at a first end 5 a connector member 7 which can be attached to connector end part 9 of the base member 3. The strap 2 furthermore comprises length adjustment means 11, not shown in detail, at which the strap 2 is looped along a buckle and fixed onto itself via a Velcro ™ fastener. At a second end 19, the strap is connected in a sliding manner to the base member 3.

After having adjusted to the right length, depending on the size of the chest of the patient, prior to first time use, the strap 2 can be closed by engagement of the first connector member 7 with the second connector member 13, which simply clicks in place. Tightening of the belt 1 can occur by one-handed operation of the sliding grip 15 and moving this sliding grip 15 towards the fixed grip 17 on the base member 3. When tightening the strap 2, the patient puts the palm of his hand against the fixed grip 17 while hooking his fingertips of 3 or 4 fingers behind the upstanding edge of the sliding grip 15 and squeezes his hand closed. Hereby the second end 19 of the strap 2 is pulled around a roller member on the base in the direction of the guide end part 21 of the base member 3 and the strap 2 is tightened around the chest of the patient.

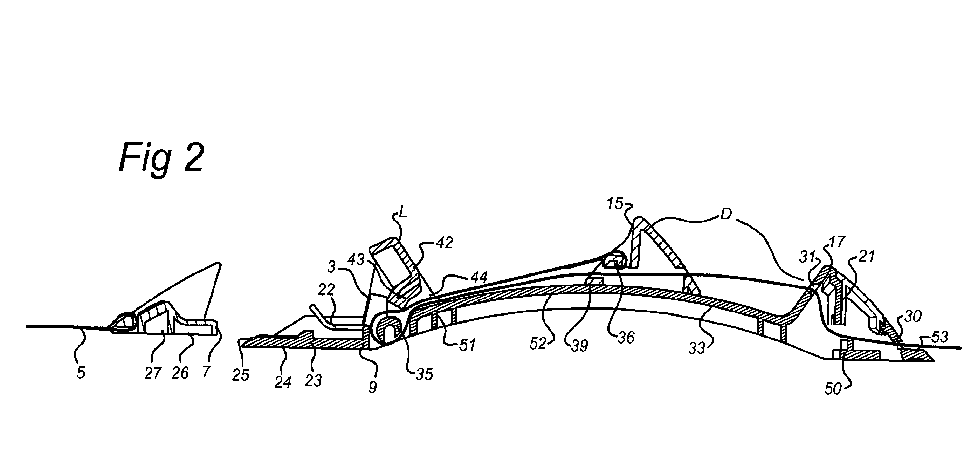

Figure 2 shows the base member 3 being provided near the connector end part 9 with first connector member 13 comprising a first guiding projection 22, a ledge 23 and a ridge 24. Upon connection, the lip 25 of the first connector member 7 can be slid under the projection 22, such that the slit 26 accommodates the ledge 23 and the sharp ledge 23 engages with the vertical side 27 of the first connector member 7.

The second end of the strap 2 is guided via slits 30,31 in the fixed grip 17 along a curved guide surface 33 to roller member 35. The roller member 35 can be a metal cylindrical pin that is fixed or that can be rotatingly connected to a support on the base member 3. The pin can have a smooth surface in case it is fixed or may be profiled when it is mounted in a rotating manner. The end section of the strap 2 is looped back along the roller member 35 in the direction of the fixed grip 17 and is attached to the sliding grip 15. The sliding grip 15 can be moved over the guide surface 33 along the strap 2 that passes through a slit 38 in the sliding grip 15, which is about the same width as the width of the strap 2.

A stop member 39 is provided on the strap 2 in order to block movement of the sliding grip 15 such that the distance D between the fixed grip 17 and the sliding grip 15 is no longer than the span of a grip of a patient, which may, depending on the size of the hands of the patient, lie between 5 and 25 cm and preferably be about 10 cm.

A pivoting lever 42 is attached in a pivot point 43 and can be pulled in the direction of the arrow L by a patient, to bring a clamping surface 44 into engagement with the strap 2 and clamps the strap against the roller member 35. After releasing the lever 42, it remains in an upright position and keeps the strap 2 in a tightened state around the patient's chest, such that the patient has two hands available for other tasks than squeezing of the strap. The tension on the strap can be quickly released by pressing down upon the lever 42.

The base member 3 is of hollow curved shape and has first and second ends 50,51 which are supported on the chest and a middle section 52 that is spaced away from a flat support surface 53 (indicated with the dashed line in the drawing) in order to prevent the base member 3 from contacting the underlying wound to the chest.

Figure 3 shows the base member 3 in an operative state in which the first and second connector members 7, 13 are coupled.

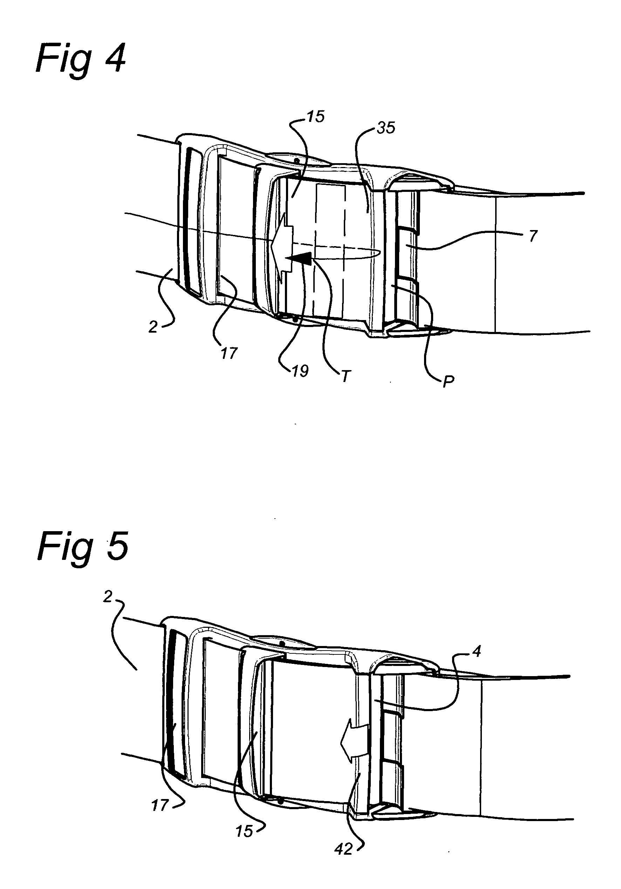

Figure 4 schematically shows how by moving the sliding grip 15 in the direction of the arrow T, the strap 2 is pulled around the roller member 35 in the direction P of the fixed grip 17, such that the belt 2 is tightened. The ergonomic design of the grips 15 and 17 allow the patient to easily hold the strap in a tightened state for a longer period of time, until the strain on the chest has been relieved.

Figure 5 schematically shows the functioning of the pivoting lever 42, which is operated by pulling it upward in the direction of the arrow L to clamp the strap 2 against the roller member 35. After releasing the lever 42 it remains in clamping engagement with the roller member 35 such that the strap 2 remains under tension even when the grips 15 and 17 are released.

## Claims

1. Chest support belt (1) for wearing around a chest of a human being, comprising a strap (2) that is, in use, connected with a first end (5) to a connector end part (9) of a base member (3), **characterised in that** a second end (19) of the strap (2) is attached to a sliding grip (15) and is guided from a guide end (21) part of the base member (3), which guide end part is situated opposite to the connector end part (9), over a guide surface (33) of the base member, in the direction of the connector end part (9) to a roller member (35), and along the roller member (35) back in the direction of the guide end part (21), wherein the sliding grip (15) can slide along the base member (3), the base member comprising near the guide end part (21) a fixed grip (17) that is fixed to the guide surface (33) of the base member (3), which fixed grip (17) in an untensioned state of the strap (2) is situated at a distance (D) of between 5 and 25 cm, preferably between 10 and 20 cm from the sliding grip (15).

2. Chest support (1) belt according to claim 1, wherein the sliding grip (15) comprises a slit (38) through which the strap (2) is guided, the slit having about the same width as the width of the strap.

3. Chest support (1) belt according to claim 1 or 2, wherein the strap (2) is guided through a second slit (30,31) in the base member (3) from the guide end part (21) towards the roller member (35).

4. Chest support belt (1) according to any of the preceding claims, wherein the base member (3) has near the connector end part (9) a first connector member (13) which cooperates with a second connector member (7) attached to the first end (5) of the strap (12).

5. Chest support (1) belt according to any of the preceding claims, the base member (3) comprising near the roller member (35) a pivoting lever (42) which can be pivoted with a clamping surface (44) into clamping engagement with the roller member (35) and/or with the guide surface (33)..

6. Chest support (1) belt according to any of the preceding claims, wherein the base member (3) has a curved surface (33) with the first and second ends (50,51) defining a support surface (53) and with a middle section (52) spaced at a distance from the support surface.

## Patentansprüche

1. Bruststützgürtel (1) zum Tragen um den Brustkorb eines Menschen, umfassend einen Gurt (2), der bei Verwendung mit einem ersten Ende (5) an einem Verbindungsstückendteil (9) eines Trägerteils (3) verbunden ist, **dadurch gekennzeichnet, dass** ein zweites Ende (19) des Gurts (2) an einem verschiebbaren Griff (15) befestigt ist und von einem Führungsendteil (21) des Trägerteils (3), wobei das Führungsendteil gegenüber dem Verbindungsstückendteil (9) liegt, über eine Führungsfläche (33) des Trägerteils in die Richtung des Verbindungsstückendteils (9) zu einem Gleitrollenelement (35) und an dem Gleitrollenelement (35) entlang zurück in die Richtung des Führungsendteils (21) geführt wird, wobei der verschiebbare Griff (15) an dem Trägerteil (3) entlang gleiten kann, das Trägerteil in der Nähe des Führungsendteils (21) einen feststehenden Griff (17) aufweist, der an der Führungsfläche (33) des Trägerteils (3) festgemacht ist, wobei der feststehende Griff (17) in einem schlaffen Zustand des Gurtes (2) in einem Abstand (D) von zwischen 5 und 25 cm, vorzugsweise zwischen 10 und 20 cm, von dem verschiebbaren Griff (15) liegt.

2. Bruststützgürtel (1) nach Anspruch 1, bei dem der verschiebbare Griff (15) einen Schlitz (38) aufweist, durch den der Gurt (2) geführt wird, wobei der Schlitz etwa die gleiche Breite besitzt wie die Breite des Gurts.

3. Bruststützgürtel (1) nach Anspruch 1 oder 2, bei dem der Gurt (2) durch einen zweiten Schlitz (30, 31) in dem Trägerteil (3) von dem Führungsendteil (21) zu dem Gleitrollenelement (35) hin geführt wird.

4. Bruststützgürtel (1) nach einem der vorhergehenden Ansprüche, bei dem das Trägerteil (3) in der Nähe des Verbindungsstückendteils (9) ein erstes Verbindungsstückelement (13) aufweist, das mit einem an dem ersten Ende (5) des Gurts (2) befestigten zweiten Verbindungsstückelement (7) zusammenwirkt.

5. Bruststützgürtel (1) nach einem der vorhergehenden Ansprüche, bei dem das Trägerteil (3) in der Nähe des Gleitrollenelements (35) einen Schwenkhebel (42) aufweist, der mit einer Klemmfläche (44) in einen Klemmeingriff mit dem Gleitrollenelement (35) und/oder mit der Führungsfläche (33) gedreht werden kann.

6. Bruststützgürtel (1) nach einem der vorhergehenden Ansprüche, bei dem das Trägerteil (3) eine gekrümmte Fläche (33) aufweist, deren erstes und zweites Ende (50, 51) eine Stützfläche (53) bilden und deren mittlerer Abschnitt (52) in einem Zwischenraum im Abstand von der Stützfläche angeordnet ist.

## Revendications

1. Ceinture thoracique (1) destinée à être portée autour du thorax d'un être humain, comprenant une sangle (2) qui est, utilisé en étant raccordée d'une première extrémité (5) à une partie d'extrémité de connecteur (9) d'un élément de base (3), **caractérisée en ce qu'**une seconde extrémité (19) de la sangle (2) est fixée à une attache coulissante (15) et est guidée à partir d'une partie d'extrémité de guidage (21) de l'élément de base (3), laquelle partie d'extrémité de guidage est située à l'opposé de la partie d'extrémité de connecteur (9) au-dessus d'une surface de guidage (33) de l'élément de base, dans le sens de la partie d'extrémité de connecteur (9) vers un élément de rouleau (35), et revient le long de l'élément de rouleau (35) dans le sens de la partie d'extrémité de guidage (21), dans laquelle l'attache coulissante (15) peut coulisser le long de l'élément de base (3), l'élément de base comprenant, à proximité de la partie d'extrémité de guidage (21), une attache fixe (17) qui est fixée à la surface de guidage (33) de l'élément de base (3), laquelle attache fixe (17), dans un état non tendu de la sangle (2), est située à une distance (D) comprise entre 5 et 25 cm, de préférence entre 10 et 20 cm de l'attache coulissante (15).

2. Ceinture thoracique (1) selon la revendication 1, dans laquelle l'attache coulissante (15) comprend une fente (38) à travers laquelle la sangle (2) est guidée, la fente possède environ la même largeur que la largeur de la sangle.

3. Ceinture thoracique (1) selon la revendication 1 ou 2, dans laquelle la sangle (2) est guidée à travers une seconde fente (30, 31) dans l'élément de base (3) à partir de la partie d'extrémité de guidage (21) vers l'élément de rouleau (35).

4. Ceinture thoracique (1) selon l'une quelconque des revendications précédentes, dans laquelle l'élément de base (3) présente, à proximité de la partie d'extrémité de connecteur (9), un premier élément de connecteur (13) qui coopère avec un second élément de connecteur (7) fixé sur la première extrémité (5) de la sangle (12).

5. Ceinture thoracique (1) selon l'une quelconque des revendications précédentes, l'élément de base (3) comprenant, à proximité de l'élément de rouleau (35), un levier pivotant (42) qui peut pivoter avec une surface de serrage (44) mis en prise de serrage avec l'élément de rouleau (35) et/ou avec la surface de guidage (33).

6. Ceinture thoracique (1) selon l'une quelconque des revendications précédentes, dans laquelle l'élément de base (3) présente une surface incurvée (33) avec les première et seconde extrémités (50, 51) définissant une surface de maintien (53) et avec une section centrale (52) espacée à une certaine distance de la surface de maintien.
